# EUROPEAN PATENT APPLICATION

(11) **EP 4 156 099 A1**
(43) Date of publication of application: **29.03.2023**
(21) Application number: 20936695.4
(22) Date of filing: 22.05.2020
(51) Int. Cl.: G06T 7/174

(54) **HANDWASHING RECOGNITION SYSTEM AND HANDWASHING RECOGNITION METHOD**

(71) Applicant: FUJITSU LIMITED, Kawasaki-shi, Kanagawa 211-8588 (JP)
(72) Inventor: IWASAKI, Sho, Kawasaki-shi, Kanagawa 211-8588 (JP); SUZUKI, Genta, Kawasaki-shi, Kanagawa 211-8588 (JP); FUJII, Yusaku, Kawasaki-shi, Kanagawa 211-8588 (JP)
(74) Representative: Hoffmann Eitle
(86) International application number: PCT/JP2020/020403
(87) International publication number: WO 2021/234972

(57) **Abstract**

The present invention provides a method to prompt a user to perform appropriate handwashing based on the state of a hand or hand fingers of the user. A handwash monitoring system includes an imaging unit (10), a detector (21), and an abnormality type determination unit (22). The detector (21) detects a first candidate abnormality existing in a hand of a user from a first image captured by the imaging unit before handwashing, and detect a second candidate abnormality existing in the hand of the user from a second image captured by the imaging unit after the handwashing. The abnormality type determination unit (22) determines a type of an abnormality on the hand of the user based on a comparison between the first candidate abnormality and the second candidate abnormality.

## Description

### Technical Field

The embodiments discussed herein are related to a system, a method, and a program for monitoring a hand-washing motion of a person.

### Background Art

In a food preparation/processing site, a pharmaceutical factory, a hospital, a nursing facility and others, hygiene management of a worker is important for prevention of infectious diseases. In particular, hand finger hygiene management including a handwash is considered to be very important because hand fingers tend to cause virus or bacteria to spread. Hazard Analysis and Critical Control Point (HACCP) requires food-related business operators to check, monitor, and record hygiene management activities. Therefore, there is a need for a technique for monitoring and recording the state of the hand fingers of the worker.

In such a situation, a handwash monitoring method including the following steps is proposed. The image acquisition step acquires hand-washing images captured by an imaging means installed in a sink for a handwash. The hand region extraction step extracts a hand region from the acquired hand-washing images to generate a frame image. The handwash start decision step decides whether a handwashing has started from the frame image. The washing method recognition step identifies the type of washing method performed by extracting the shape of the hand region from the frame image. The scrubbing decision step decides whether the scrubbing state about the washing method is good or bad by adding the number of seconds to the washing method when the identified washing method is a washing method of specified order. The handwash end decision step decides the end of the handwashing from the frame image (For example, Patent Document 1).

In addition, there has been proposed a hygiene management device capable of automatically determining whether a handwashing is correctly performed and preventing entry from a hand finger washing area to a hygiene management area unless a handwashing is correctly performed (e.g., refer to Patent Document 2). There has been proposed a method capable of detecting a wide range of motion information in a non-contact manner and analyzing a hand finger hygiene behavior in real time (e.g., refer to Patent Document 3). There has been proposed a handwash monitoring system capable of improving recognition accuracy of a person who washes hands (e.g., refer to Patent Document 4).

### List of Prior Art Documents

### Patent Documents

Patent Document 1: Japanese Laid-Open Patent Publication No. 2017-134712
Patent Document 2: Japanese Laid-Open Patent Publication No. 2002-085271
Patent Document 3: Japanese Laid-Open Patent Publication No. 2018-117981
Patent Document 4: Japanese Laid-Open Patent Publication No. 2020-018674

### Summary of the Invention

### Problem to be Solved by the Invention

In a conventional handwash monitoring method, the state of a hand or hand finger may not be accurately determined. For example, dirt, a wound, a mole, a tattoo, or others may not be correctly identified. If the state of the hand or hand finger is not correctly determined, it is impossible to determine whether a handwashing has been appropriately performed. It is also difficult to give an appropriate instruction to a worker. For example, in a case where a "wound" is erroneously recognized as "dirt", it is determined that dirt remains even if an appropriate handwashing is performed. In this case, despite the absence of dirt, a message is output to the worker to continue the handwashing.

An object of one aspect of the present invention is to prompt a user to perform appropriate handwashing based on the state of a hand or hand fingers of the user.

### Means for Solving the Problems

A handwash monitoring system according to one aspect of the present invention includes: an imaging unit; a detector configured to detect a first candidate abnormality existing in a hand of a user from a first image captured by the imaging unit before handwashing, and detect a second candidate abnormality existing in the hand of the user from a second image captured by the imaging unit after the handwashing; and an abnormality type determination unit configured to determine a type of an abnormality on the hand of the user based on a comparison between the first candidate abnormality and the second candidate abnormality.

### Effects of Invention

According to the above-described aspect, the user is prompted to perform appropriate handwashing based on the state of the hand or hand fingers of the user.

### Brief Description of Drawings

FIG. 1 illustrates an example of a handwash monitoring system according to an embodiment of the present invention.
FIG. 2 is a flowchart illustrating an example of a process of the handwash monitoring system.
FIG. 3 illustrates an example of a method of detecting a candidate abnormality.
FIG. 4 illustrates an example of a method of determining a type of an abnormality.
FIG. 5 is a flowchart illustrating an example of a process for determining a type of an abnormality.
FIG. 6 is a flowchart illustrating a first variation of a handwash monitoring method.
FIG. 7 illustrates an example of a method of detecting a candidate abnormality using a reference hand finger image.
FIG. 8 illustrates an example of a candidate abnormality detector used in the first variation.
FIG. 9 is a flowchart illustrating a second variation of the handwash monitoring method.
FIG. 10 is a flowchart illustrating a third variation of the handwash monitoring method.
FIG. 11 is a diagram illustrating an example of a hand-washing steps.
FIG. 12 illustrates an example of a method of determining a priority step during a handwashing.
FIG. 13 illustrates a method of detecting a state of a nail.

### Embodiments of the Invention

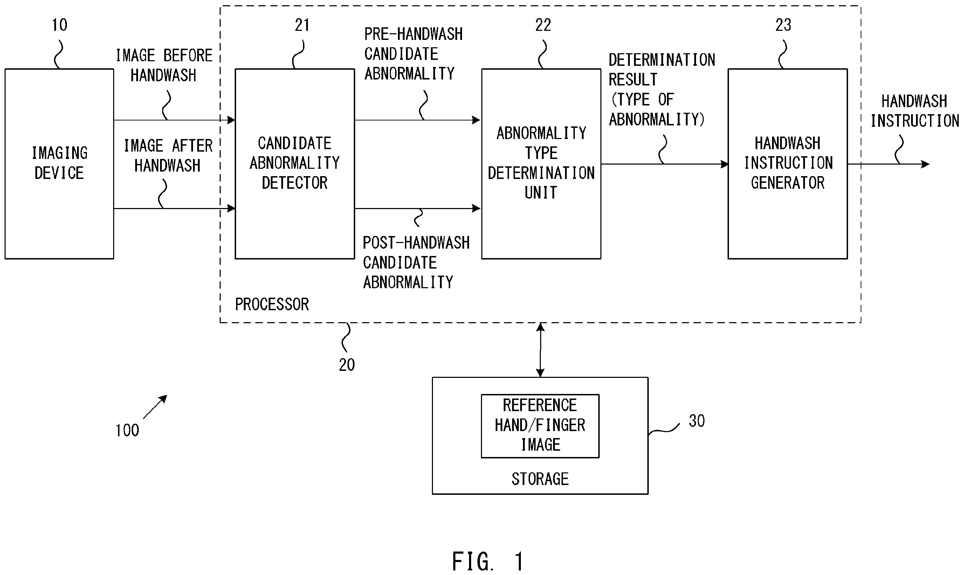

FIG. 1 illustrates an example of a handwash monitoring system according to an embodiment of the present invention. As illustrated in FIG. 1, a handwash monitoring system 100 according to the embodiment of the present invention includes an imaging device 10, a candidate abnormality detector 21, an abnormality type determination unit 22, a handwash instruction generator 23, and a storage 30. Note that the handwash monitoring system 100 may include other functions or devices not illustrated in FIG. 1.

The imaging device 10 is, for example, a digital camera, and acquires a color image by photographing. The imaging device 10 may acquire an image at a specified time interval (e.g., 30 frames/sec) . In this case, the imaging device 10 can substantially acquire a moving picture. The imaging device 10 is installed above a sink where a person washes his or her hands, for example. The imaging device 10 then photographs the hand fingers of a person during a handwashing. Note that, in the following description, a "hand/finger" is assumed to mean not only "fingers of a hand" but also a "hand including fingers of the hand" or "hand and hand fingers". In the following description, a person whose hand/finger is imaged by the imaging device 10 may be referred to as a "user". The user is not particularly limited, but is, for example, a worker at a food preparation/processing site, a pharmaceutical factory, a hospital, a nursing facility, or others.

The handwash monitoring system 100 generates an instruction related to a handwashing by using an image captured by the imaging device 10 and provides the instruction to the user. For example, if the user wears an accessory (a ring, a watch, or others) on his or her hand/finger, the handwash monitoring system 100 may instruct the user to remove the accessory and perform handwashing. If dirt remains after handwashing, the handwash monitoring system 100 may instruct the user to perform handwashing again. Further, if the hand/finger of the user has a wound, the handwash monitoring system 100 may instruct the user to treat the wound with an adhesive plaster or others and/or to wear vinyl gloves. Note that, in the following description, an instruction related to a handwashing provided to the user may be referred to as a "handwash instruction".

However, simple image recognition may not be able to accurately identify the state of a hand/finger. If the state of the hand/finger is not correctly identified, the handwash monitoring system 100 cannot provide an appropriate handwash instruction to the user. Therefore, the handwash monitoring system 100 identifies the state of the hand/finger of the user by using the image of the hand/finger before handwashing and the image of the hand/finger after handwashing.

The candidate abnormality detector 21 detects a candidate abnormality existing in the hand/finger of a user from an image captured by the imaging device 10 before handwashing. The candidate abnormality detector 21 detects a candidate abnormality existing in the hand/finger of a user from an image captured by the imaging device 10 after the handwashing. The "abnormality" includes dirt, a wound, and an accessory. Therefore, the "candidate abnormality" corresponds to an image region that may be determined to be an "abnormality" in image recognition. As an example, in a hand region extracted from an input image, a region including a color component different from a general color of a hand is detected as a candidate abnormality. Note that, in the following description, a candidate abnormality detected from an image captured by the imaging device 10 before handwashing may be referred to as a "pre-handwash candidate abnormality (first candidate abnormality) ". A candidate abnormality detected from an image captured by the imaging device 10 after handwashing may be referred to as a "post-handwash candidate abnormality (second candidate abnormality)".

The abnormality type determination unit 22 determines the type of the abnormality on the hand/finger of the user based on the pre-handwash candidate abnormality and the post-handwash candidate abnormality detected by the candidate abnormality detector 21. Here, the abnormality type determination unit 22 can determine the type of the abnormality on the hand/finger of the user based on the comparison between the pre-handwash candidate abnormality and the post-handwash candidate abnormality. The handwash instruction generator 23 generates a handwash instruction based on the type of the abnormality on the hand/finger of the user determined by the abnormality type determination unit 22. The generated handwash instruction is provided to the user. The handwash instruction generator 23 may generate a warning sound based on the type of the abnormality on the hand/finger of the user determined by the abnormality type determination unit 22 to prompt the user to perform handwashing. The handwash operation corresponding to the warning sound may be communicated to the user by means other than the handwash monitoring system 100, for example, by a notice.

The candidate abnormality detector 21, the abnormality type determination unit 22, and the handwash instruction generator 23 are realized by a processor 20 executing a handwash monitoring program. In other words, the functions of the candidate abnormality detector 21, the abnormality type determination unit 22, and the handwash instruction generator 23 are provided by the processor 20 that executes the handwash monitoring program. In this case, the handwash monitoring program is stored in, for example, the storage device 30. Alternatively, the processor 20 may execute a handwash monitoring program recorded on a removable recording medium not illustrated. Further, the processor 20 may acquire a handwash monitoring program from a program server not illustrated and execute the handwash monitoring program.

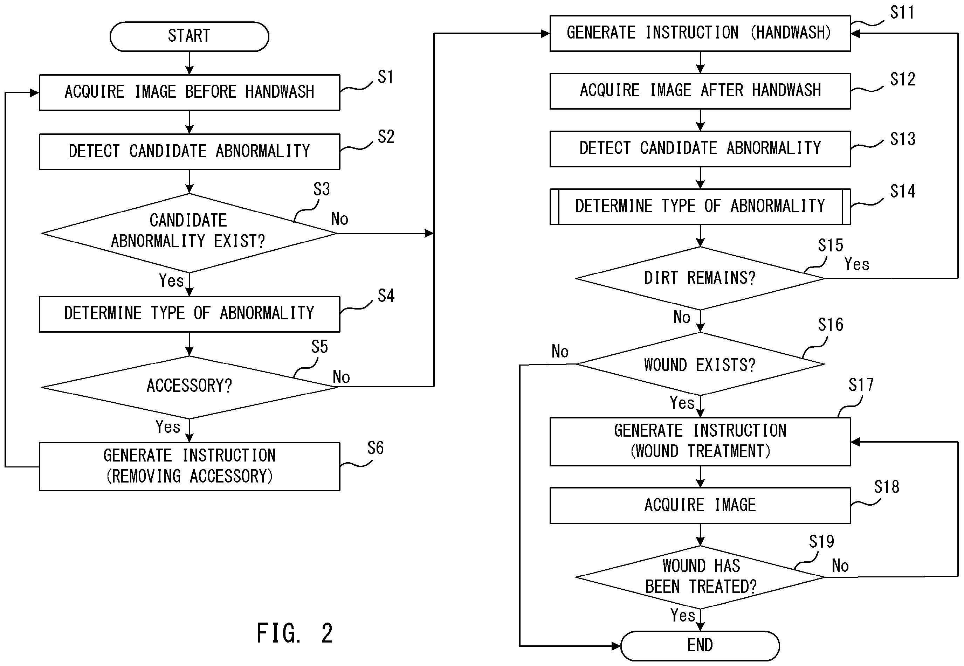

FIG. 2 is a flowchart illustrating an example of a process of the handwash monitoring system 100. Note that the handwash monitoring process is executed, for example, when a start instruction is given from the user. Alternatively, the handwash monitoring process is executed when the user is positioned at a specified location (e.g., in front of a handwash sink).

In S1, the processor 20 acquires an image of the hand/finger of the user before handwashing. The processor 20 preferably acquires an image from the wrist to the fingertip. The processor 20 preferably acquires an image of the palm side and an image of the back side of the hand. Therefore, although not particularly limited, the handwash monitoring system 100 may provide the user with guidance indicating that the image of the palm including the hand/finger and the wrist and the image of the back of the hand including the hand/finger and the wrist are necessary.

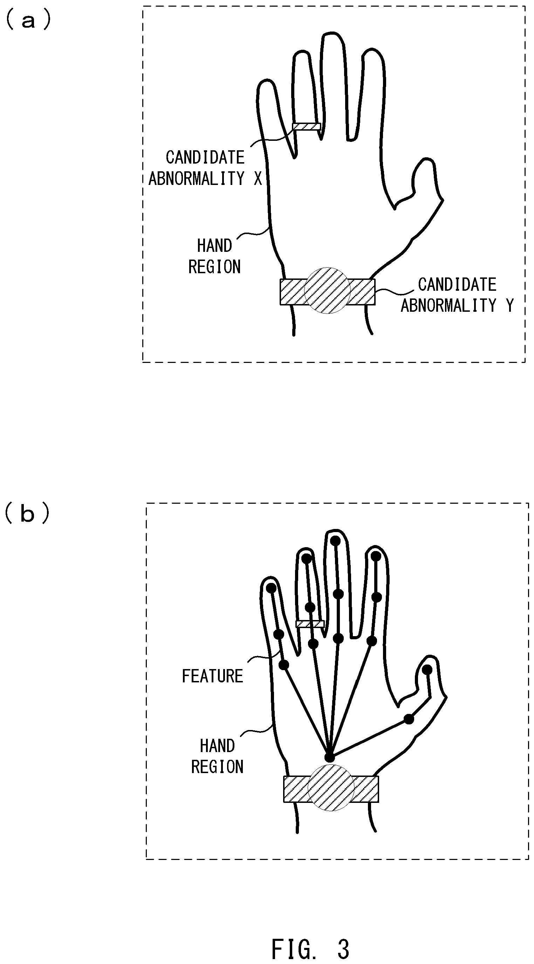

In S2-S3, the candidate abnormality detector 21 detects a candidate abnormality (i.e., a pre-handwash candidate abnormality) from the input image. During this step, the candidate abnormality detector 21 extracts a hand region corresponding to the hand of the user from the input image. A method of extracting a hand region from the input image is not particularly limited, and is realized by a known technique. For example, the candidate abnormality detector 21 may extract the hand region from the input image by using semantic segmentation. The candidate abnormality detector 21 then detects a candidate abnormality existing in the hand region. As an example, a region including a color component different from a general color of a hand in the hand region is detected as a candidate abnormality. The candidate abnormality detector 21 may detect a candidate abnormality by image recognition deep learning, for example. In the example illustrated in FIG. 3 (a), a candidate abnormality X and a candidate abnormality Y are detected.

If one or more candidate abnormalities are detected, in S4-S5, the abnormality type determination unit 22 determines the type of the abnormality for each candidate abnormality. During this step, the abnormality type determination unit 22 determines, for example, whether the detected candidate abnormality corresponds to "dirt", "wound", or "accessory". The type of the abnormality is determined by, for example, image recognition deep learning. As an example, as illustrated in FIG. 3(b), the abnormality type determination unit 22 estimates the posture of the hand/finger by acquiring the feature information of the hand region. Thus, the position of each candidate abnormality with respect to the hand/finger of the user is specified. Specifically, the candidate abnormality X is estimated to be positioned at the base of the ring finger. In this case, the abnormality type determination unit 22 determines that the candidate abnormality X is an accessory (ring). The candidate abnormality Y is estimated to be positioned at the wrist. In this case, the abnormality type determination unit 22 determines that the candidate abnormality Y is an accessory (wrist watch).

Note that the abnormality type determination unit 22 may determine the type of the abnormality in consideration of the shape and/or the size of the candidate abnormality in addition to the position of the candidate abnormality. The abnormality type determination unit 22 may determine the type of the abnormality without estimating the posture of the hand/finger. For example, the abnormality type determination unit 22 may determine the type of the abnormality based on the size and the shape of the candidate abnormality.

If the candidate abnormality is an accessory, in S6, the handwash instruction generator 23 generates a handwash instruction for requesting removal of the accessory from the hand. The handwash instruction is provided to the user. The handwash instruction is realized by, for example, a voice message. Alternatively, if the handwash monitoring system 100 includes a display device, the handwash instruction may be displayed on the display device.

The process of the handwash monitoring system 100 then returns to S1. In other words, the handwash monitoring system 100 again acquires an image of the hand/finger of the user before handwashing. If it is confirmed that the accessory has been removed from the hand of the user (S5: No), the process of the handwash monitoring system 100 proceeds to S11. Note that, even if the candidate abnormality is not detected in S3, the process of the handwash monitoring system 100 proceeds to S11.

In S11, the handwash instruction generator 23 generates a handwash instruction for requesting to perform handwashing. The handwash instruction is also provided to the user as in S6.

In S12, the processor 20 acquires an image of the hand/finger of the user after handwashing. Also in S12, as in S1, the processor 20 preferably acquires an image from the wrist to the fingertip. The processor 20 preferably acquires an image of the palm side and an image of the back side of the hand. In other words, the processor 20 preferably acquires images of the identical hand/finger posture before handwashing and after handwashing.

In S13, the candidate abnormality detector 21 detects a candidate abnormality (i.e., post-handwash candidate abnormality) from the input image. A method of detecting a candidate abnormality from the input image is substantially identical in S2 and S13. In other words, the candidate abnormality is detected by, for example, image recognition deep learning.

In S14, the abnormality type determination unit 22 determines the type of the abnormality. During this step, the abnormality type determination unit 22 determines the type of the abnormality for each candidate abnormality detected from the input image after handwashing. In addition, the abnormality type determination unit 22 determines the type of the abnormality based on the pre-handwash candidate abnormality and the post-handwash candidate abnormality. Specifically, the abnormality type determination unit 22 determines the type of the abnormality based on a comparison between the pre-handwash candidate abnormality and the post-handwash candidate abnormality.

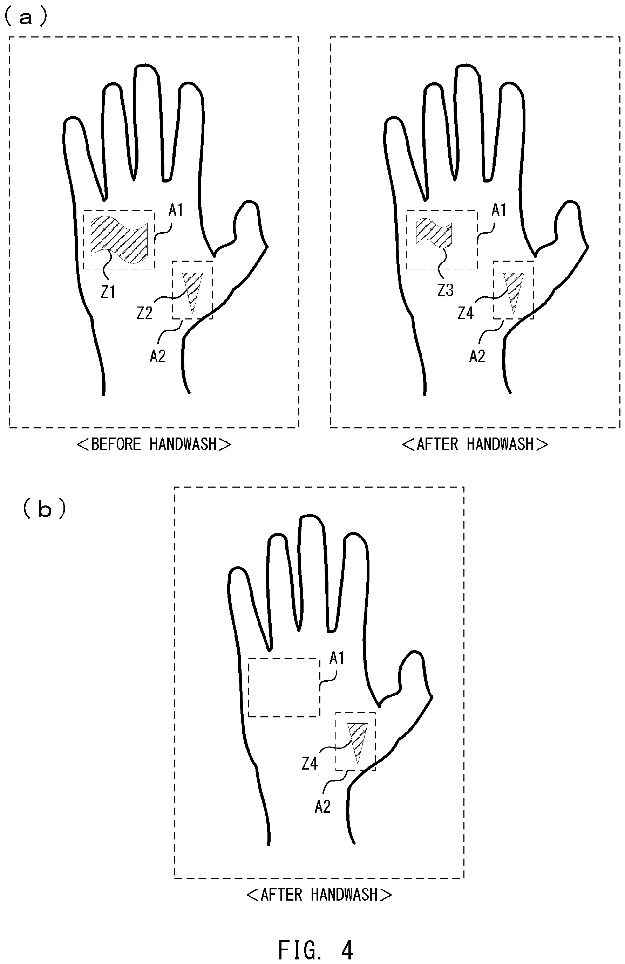

In the example illustrated in FIG. 4(a), a candidate abnormality Z1 and a candidate abnormality Z2 are detected in the image of the hand/finger of the user before handwashing. A candidate abnormality Z3 is detected in the image of the hand/finger of the user after handwashing. The abnormality type determination unit 22 performs hand/finger posture estimation and size normalization for each input image. The hand/finger posture estimation estimates the posture of the hand/finger by analyzing the shape of the hand/finger of the user. The size normalization refers to the result of the hand/finger posture estimation, and adjusts the size of one or both of the two input images so that the sizes of the hand regions extracted from the two input images match each other. The abnormality type determination unit 22 then compares the pre-handwash candidate abnormality with the post-handwash candidate abnormality by comparing the two input images.

In FIG. 4(a), the candidate abnormality Z1 and the candidate abnormality Z2 are detected from a region A1 and a region A2 of the input image before handwashing, respectively. The candidate abnormality Z3 and the candidate abnormality Z4 are detected from the region A1 and the region A2 of the input image after handwashing, respectively. In other words, the candidate abnormality Z1 and the candidate abnormality Z3 are detected from the identical region A1 in the hand region, and the candidate abnormality Z2 and the candidate abnormality Z4 are detected from the identical region A2 in the hand region.

"Dirt" attached to the hand/finger of the user is removed by handwashing. Therefore, when the candidate abnormalities detected from the identical region are compared with each other, if the shape of the candidate abnormality detected from the image after handwashing (i.e., the post-handwash candidate abnormality) is different from the shape of the candidate abnormality detected from the image before handwashing (i.e., the pre-handwash candidate abnormality), the candidate abnormality is determined to be "dirt".

For example, if the size of the post-handwash candidate abnormality is smaller than the size of the pre-handwash candidate abnormality in the identical region, the candidate abnormality is determined to be "dirt". In the example illustrated in FIG. 4 (a), the candidate abnormality Z1 and the candidate abnormality Z3 are detected from the identical region A1, and the size of the candidate abnormality Z3 is smaller than the size of the candidate abnormality Z1. In this case, the abnormality type determination unit 22 determines that the candidate abnormalities Z1 and Z3 are "dirt" attached to the hand of the user. If handwashing is appropriately performed, the "dirt" should not exist in the input image after the handwashing. For example, in the example illustrated in FIG. 4(b), no candidate abnormality exists in the region A1 in which the candidate abnormality Z1 has been detected before handwashing. Also in this case, the abnormality type determination unit 22 determines that the candidate abnormality Z1 is "dirt".

On the other hand, the "wound" occurred in the hand/finger of the user is not removed by handwashing. Therefore, when the candidate abnormalities detected from the identical region are compared with each other, if the shape of the candidate abnormality detected from the image before handwashing (i.e., the pre-handwash candidate abnormality) and the shape of the candidate abnormality detected from the image after handwashing (i.e., the post-handwash candidate abnormality) are identical or substantially identical to each other, the candidate abnormality is determined to be a "wound". In the example illustrated in FIG. 4(a), the candidate abnormality Z2 and the candidate abnormality Z4 are detected from the identical region A2, and the shape of the candidate abnormality Z2 and the shape of the candidate abnormality Z4 are substantially identical to each other. In this case, the abnormality type determination unit 22 determines that the candidate abnormalities Z2 and Z4 are "wounds" occurred in the hand of the user.

In S15, the handwash instruction generator 23 determines whether or not "dirt" remains on the hand/finger of the user. If a candidate abnormality is detected from the identical region of the image before handwashing and the image after handwashing, and the size of the post-handwash candidate abnormality is smaller than the size of the pre-handwash candidate abnormality, it is determined that "dirt" remains on the hand/finger of the user. For example, in the case illustrated in FIG. 4(a), since the size of the candidate abnormality Z3 detected from the image after handwashing is smaller than the size of the candidate abnormality Z1 detected from the image before handwashing, it is determined that "dirt" remains in the region A1. In this case, the process of the handwash monitoring system 100 returns to S11. In other words, the handwash instruction generator 23 generates a handwash instruction for requesting to perform handwashing. Note that, in a case where S11 is performed after S12-S14 are performed, the handwash instruction may include an instruction to change a detergent to be used for handwashing. For example, the handwash instruction generator 23 may estimate that dirt that cannot be removed by normal soap is attached to the hand of the user, and recommend the use of medicinal soap having a strong cleansing power.

If no "dirt" remains on the hand/finger of the user, the process of the handwash monitoring system 100 proceeds to S16. For example, although the candidate abnormality Z1 is detected from the region A1 on the image before handwashing as illustrated in FIG. 4(a), no candidate abnormality exists in the identical region A1 on the image after handwashing as illustrated in FIG. 4(b). In this case, the handwash instruction generator 23 determines that "dirt" is removed and does not remain in the region A1. Note that, even if there is no candidate abnormality determined to be "dirt" in S13-S14, the process of the handwash monitoring system 100 proceeds to S16.

In S16, the handwash instruction generator 23 determines whether or not a "wound" occurs in the hand/finger of the user. In the case illustrated in FIG. 4(a), the candidate abnormalities Z2 and Z4 are determined to be "wounds". If a "wound" occurs in the hand/finger of the user, in S17, the handwash instruction generator 23 generates a handwash instruction for requesting a treatment of the wound. The handwash instruction includes, for example, an instruction to stick an adhesive plaster and an instruction to wear vinyl gloves. The handwash instruction includes a message indicating that an image of a hand/finger whose wound treatment is completed needs to be captured. Note that the handwash instruction is also provided to the user as in S6.

In S18, the processor 20 acquires an image of the hand/finger of the user. During this step, the processor 20 acquires an image including a region in which a wound occurs. In S19, the handwash instruction generator 23 determines whether the wound on the hand/finger of the user has been appropriately treated based on the image acquired in S18. For example, if an image corresponding to an "adhesive plaster" is detected in a region determined to be a "wound", the handwash instruction generator 23 determines that the wound on the hand/finger of the user has been appropriately treated. In this case, the process of the handwash monitoring system 100 is ended. Note that, if the process of the flowchart illustrated in FIG. 2 is ended, the handwash monitoring system 100 may record on a server computer that the hygienic state of the hand/finger of the user is good. On the other hand, if it is not determined that the wound on the hand/finger of the user has been appropriately treated, the process of the handwash monitoring system 100 returns to S17.

As described above, if dirt remains on the hand/finger of the user, the process of the flowchart illustrated in FIG. 2 is not ended. In this case, S11-S15 are repeatedly performed. If a wound occurs in the hand/finger of the user, the process of the flowchart illustrated in FIG. 2 is not ended until the wound is appropriately treated. In this case, S17-S19 are repeatedly performed. Therefore, according to the embodiment of the present invention, it is possible to appropriately manage the hygienic state of the hand/finger of the user.

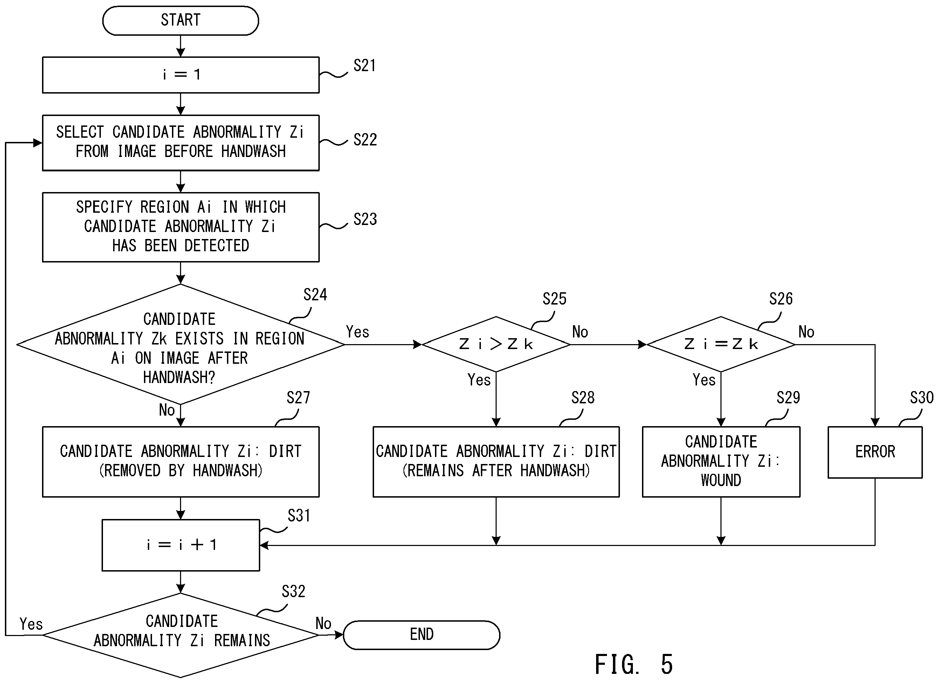

FIG. 5 is a flowchart illustrating an example of a process of determining a type of an abnormality. This process corresponds to S14 illustrated in FIG. 2. In other words, the process of this flowchart is executed by the abnormality type determination unit 22 after the process of detecting the candidate abnormality from the image before handwashing and the process of detecting the candidate abnormality from the image after handwashing.

In S21, the abnormality type determination unit 22 initializes a variable i. The variable i identifies the candidate abnormality detected from the image before handwashing. In the example illustrated in FIG. 4(a), the candidate abnormalities Z1 and Z2 are identified.

In S22, the abnormality type determination unit 22 selects a candidate abnormality Zi from among the candidate abnormalities detected from the image before handwashing. In S23, the abnormality type determination unit 22 specifies a region in which the candidate abnormality Zi has been detected. In the description related to FIG. 5, the region in which the candidate abnormality Zi has been detected may be referred to as a "region Ai".

In S24, the abnormality type determination unit 22 determines whether a candidate abnormality exists in the region Ai on the image after handwashing. If no candidate abnormality exists in the region Ai on the image after handwashing, in S27, the abnormality type determination unit 22 determines that the candidate abnormality Zi is "dirt" and has been removed by hand wash.

If a candidate abnormality Zk exists in the region Ai on the image after handwashing, in S25-S26, the abnormality type determination unit 22 compares the candidate abnormality Zi with the candidate abnormality Zk. If the size of the candidate abnormality Zk is smaller than the size of the candidate abnormality Zi, in S28, the abnormality type determination unit 22 determines that the candidate abnormality Zi is "dirt" and that a part of the dirt has been removed by handwashing. If the candidate abnormality Zi and the candidate abnormality Zk are identical or substantially identical to each other, in S29, the abnormality type determination unit 22 determines that the candidate abnormality Zi is a "wound". If the size of the candidate abnormality Zk is larger than the size of the candidate abnormality Zi, in S30, the abnormality type determination unit 22 executes error process.

However, the abnormality type determination unit 22 may determine that the candidate abnormality Zi is "dirt" if the shape of the candidate abnormality Zi and the shape of the candidate abnormality Zk are different from each other regardless of the sizes of the candidate abnormalities Zi and Zk. For example, when dirt is attached to the hand of the user and the user rubs his or her hand against the hand during handwashing, a dirt region may be widened. In this case, even if the size of the candidate abnormality Zk is larger than the size of the candidate abnormality Zi, the candidate abnormality Zi may be determined as "dirt".

In S31, the variable i is incremented. In S32, the abnormality type determination unit 22 determines whether there remains a candidate abnormality Zi for which the process in S23-S30 has not been executed. If such a candidate abnormality Zi remains, the process of the abnormality type determination unit 22 returns to S22. In other words, the next candidate abnormality is selected from the image before handwashing. Therefore, the type of the abnormality is determined for each candidate abnormality Zi detected from the image before handwashing.

As described above, in the handwash monitoring method according to the embodiment of the present invention, the handwash flow to be performed by the user is instructed based on the change from the image before handwashing to the image after handwashing. The handwash monitoring system 100 then instructs an appropriate handwash flow and a flow after handwashing so that the hand/finger of the user (here, the worker) is in a state suitable for work, by identifying dirt, a wound, and an accessory. The method of using the change from the image before handwashing to the image after handwashing can accurately determine the type of the abnormality on the hand/finger of the user compared to simple image recognition. Therefore, the handwash monitoring method according to the embodiment of the present invention can provide an appropriate handwash instruction to the user. Thus, for example, the hygienic state of a worker in food-related businesses is improved.

### Variation 1

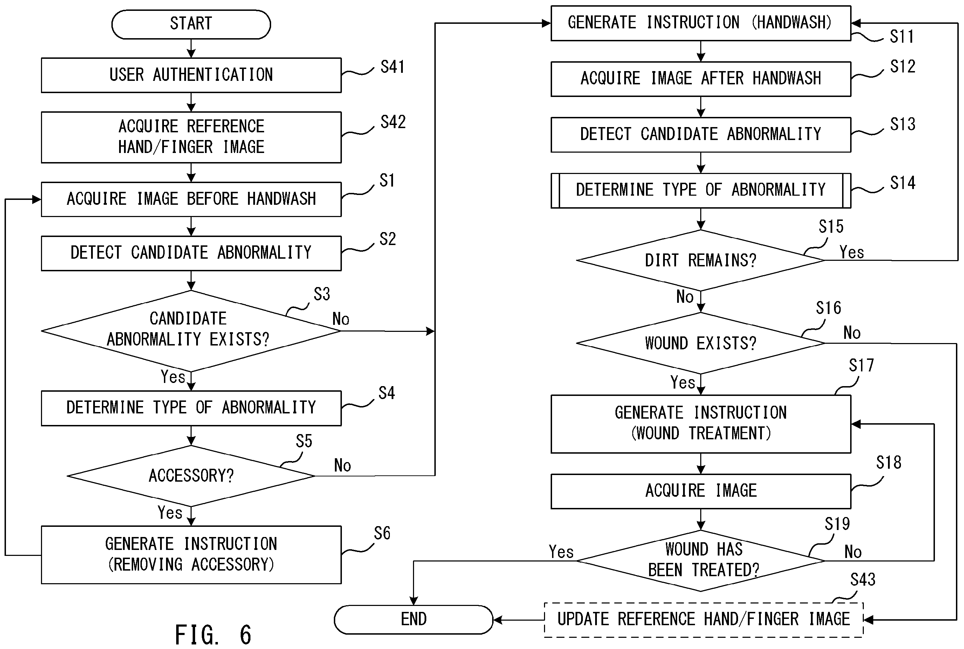

FIG. 6 is a flowchart illustrating a first variation of a handwash monitoring method. Note that, in the first variation, in addition to S1-S6 and S11-S19 illustrated in FIG. 2, S41-S43 are performed.

In S41, the handwash monitoring system 100 specifies a user. The user is specified by a known technique. For example, the user is specified by biometric authentication (face authentication, vein authentication, fingerprint authentication, iris authentication, or others). When each user holds a device (IC chip or others) for identifying an individual, the user is specified by the device. In any case, it is preferable that the user is specified in a non-contact manner.

In S42, the processor 20 acquires a reference hand/finger image of the specified user from the storage 30. Note that, as illustrated in FIG. 1, the reference hand/finger image of each user is stored in the storage 30. In this example, the reference hand/finger image is an image of the hand/finger of the user captured at a normal situation. The reference hand/finger image may be captured by the imaging device 10 or may be captured by another imaging device. As the reference hand/finger image, an image satisfying the following conditions is preferably stored.
(1) No accessory is worn on the hand/finger.
(2) No dirt is attached on the hand/finger.
(3) No wound is on the hand/finger.

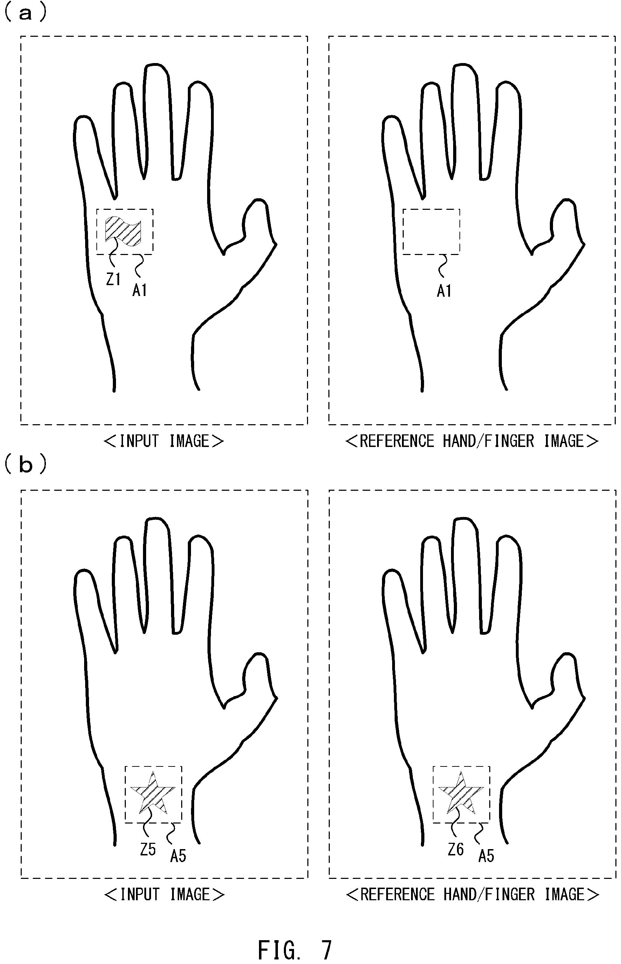

In this example, the reference hand/finger image is used in the process of detecting a candidate abnormality (S2 and S13 in FIG. 6). For example, it is assumed that the processor 20 acquires the input image and the reference hand/finger image illustrated in FIG. 7(a) . The input image is captured by the imaging device 10. The reference hand/finger image is stored in the storage 30. In this example, a candidate abnormality Z1 is detected from a region A1 on the input image. However, no candidate abnormality exists in the region A1 on the reference hand/finger image. In this case, it is estimated that the candidate abnormality Z1 is a foreign object that does not normally exist in the hand/finger of the user.

On the other hand, in the example illustrated in FIG. 7(b), a candidate abnormality Z5 is detected from a region A5 on the input image. A candidate abnormality Z6 exists in the region A5 on the reference hand/finger image. The shapes of the candidate abnormality Z5 and the candidate abnormality Z6 are identical to each other. In this case, it is estimated that the candidate abnormality Z5 detected from the input image exists in the hand/finger of the user from the normal situation. In other words, it is estimated that the candidate abnormality Z5 is not "dirt" removed by handwashing and is not a "wound" occurring in the hand/finger of the user. Therefore, the candidate abnormality Z5 is excluded from candidate abnormalities in the subsequent process. The candidate abnormality detected from the reference hand/finger image is a foreign object that exists in the hand/finger of the user from the normal situation, and corresponds to, for example, a mole, a scar, a tattoo, or others.

As described above, even if a candidate abnormality is detected from the input image, if the candidate abnormality also exists in the reference hand/finger image, it is not necessary to remove the candidate abnormality by handwashing, and it is also not necessary to perform a treatment. Such a candidate abnormality is excluded in the process in S14-S19. Therefore, in the variation 1, generation of an inappropriate handwash instruction is suppressed. For example, if a mole, a scar, a tattoo, or others is determined to be "dirt", continuation of handwashing is requested. If a mole, a scar, a tattoo or others is determined to be a "wound", a specified treatment is requested. On the other hand, in the variation 1, generation of such an inappropriate handwash instruction is suppressed.

In the process in S11-S19 illustrated in FIG. 6, if it is determined that there is no wound on the hand/finger, the processor 20 may update the reference hand/finger image in S43. For example, if the reference hand/finger image stored in the storage 30 is old, the processor 20 may store an image newly captured by the imaging device 10 in the storage 30.

In S2, the candidate abnormality detector 21 may detect a candidate abnormality by comparing the input image before handwashing with the reference hand/finger image. In S13, the candidate abnormality detector 21 may detect a candidate abnormality by comparing the input image after handwashing with the reference hand/finger image.

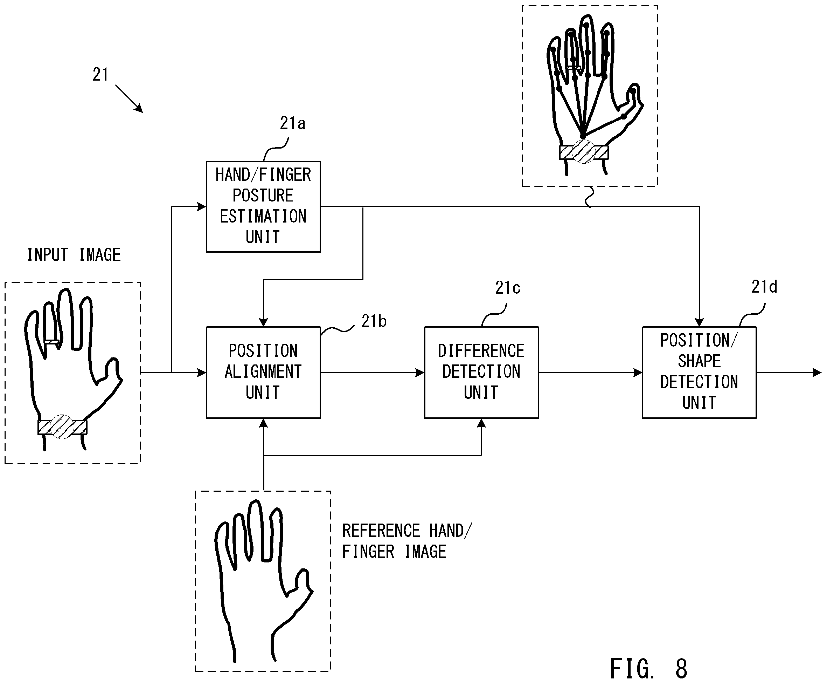

FIG. 8 illustrates an example of the candidate abnormality detector 21 used in the first variation. In this example, the candidate abnormality detector 21 includes a hand/finger posture estimation unit 21a, a position alignment unit 21b, a difference detection unit 21c, and a position/shape detection unit 21d.

The hand/finger posture estimation unit 21a estimates the posture of the hand/finger of the user by detecting the feature of the hand region extracted from the input image. The position alignment unit 21b performs position alignment between the input image and the reference hand/finger image by using non-rigid transformation based on the posture estimated by the hand/finger posture estimation unit 21a. The position alignment may be performed using wrinkles of the hand/finger or others. The difference detection unit 21c detects a difference between the input image and the reference hand/finger image. The difference region is detected as a candidate abnormality. The position/shape detection unit 21d detects the position and shape of the candidate abnormality with reference to the posture estimated by the hand/finger posture estimation unit 21a. In this example, candidate abnormalities are detected at the base of the ring finger and the wrist. Note that, in addition to the reference hand/finger image, the handwash monitoring system 100 may store accessory information representing a feature of a specified accessory in the storage 30. In this case, the accessory information preferably includes an image of a specified accessory. In S4-S5, the abnormality type determination unit 22 may determine whether the user wears an accessory on the hand/finger based on the input image and the accessory information. If it is determined that the user wears the accessory on his or her hand/finger, the handwash instruction generator 23 generates and outputs a handwash instruction for requesting removal of the accessory.

The handwash monitoring system 100 may detect a first candidate abnormality from the reference hand/finger image stored in the storage 30, and detect a second candidate abnormality from the image captured by the imaging device 10 after handwashing. In this case, the handwash instruction generator 23 generates an instruction related to a handwashing based on the comparison between the first candidate abnormality and the second candidate abnormality.

### Variation 2

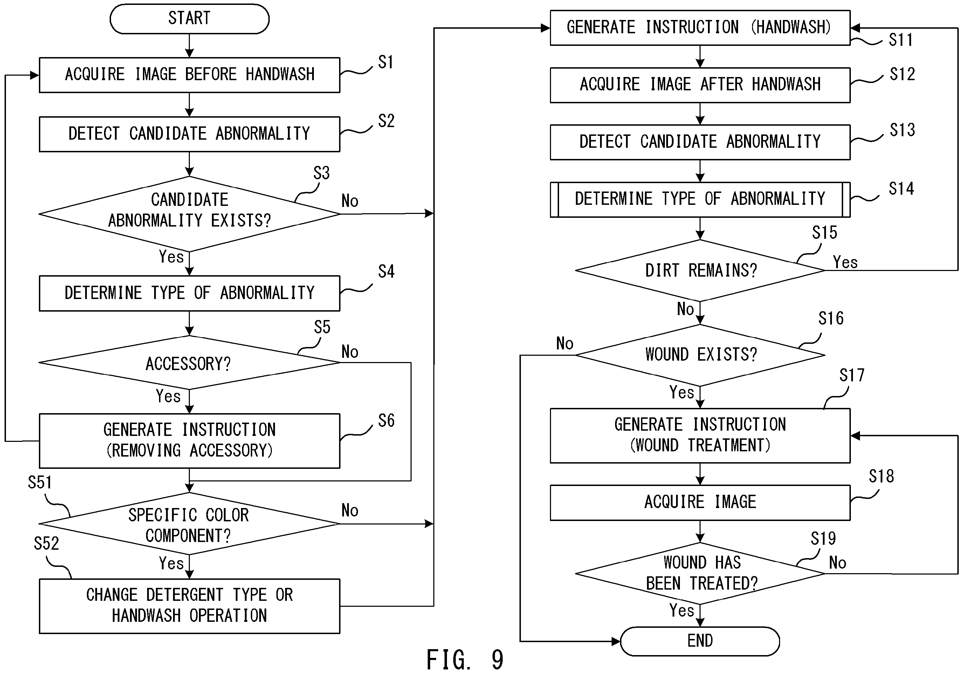

FIG. 9 is a flowchart illustrating a second variation of the handwash monitoring method. Note that, in the second variation, in addition to S1-S6 and S11-S19 illustrated in FIG. 2, S51-S52 are performed. S51-S52 are performed after S1-S6. Specifically, if it is determined that the candidate abnormality detected from the input image is not an accessory, S51-S52 are performed.

In S51-S52, the abnormality type determination unit 22 determines whether the candidate abnormality detected in S2 has a specific color component. If the candidate abnormality has a specific color component, the handwash instruction generator 23 changes the handwash flow. Specifically, if the candidate abnormality has a specific color component, the handwash instruction generator 23 may change the type of detergent and/or the handwash operation. For example, the abnormality type determination unit 22 determines whether the candidate abnormality has a color component corresponding to a specific oil stain. If the candidate abnormality has a color component corresponding to a specific oil stain, the handwash instruction generator 23 determines a detergent suitable for removing the oil stain. The handwash instruction generator 23 may determine a handwash operation suitable for removing the oil stain.

If the detergent and the handwash operation are determined in S52, in S11, the handwash instruction generator 23 generates a handwash instruction according to the determination. In this case, the handwash instruction generator 23 instructs the user to use the determined detergent. Alternatively, the handwash instruction generator 23 instructs the user to perform the determined handwash operation. Therefore, the user can sufficiently remove dirt on the hand/finger.

Note that, in the example illustrated in FIG. 9, the handwash flow is determined based on the color component of the pre-handwash candidate abnormality, but in the second variation, the handwash flow may be changed based on the color component of the candidate abnormality remaining after handwashing. For example, if it is determined that dirt remains in S15, the handwash instruction generator 23 may change the handwash flow based on the color component of the remaining candidate abnormality (i.e., dirt).

### Variation 3

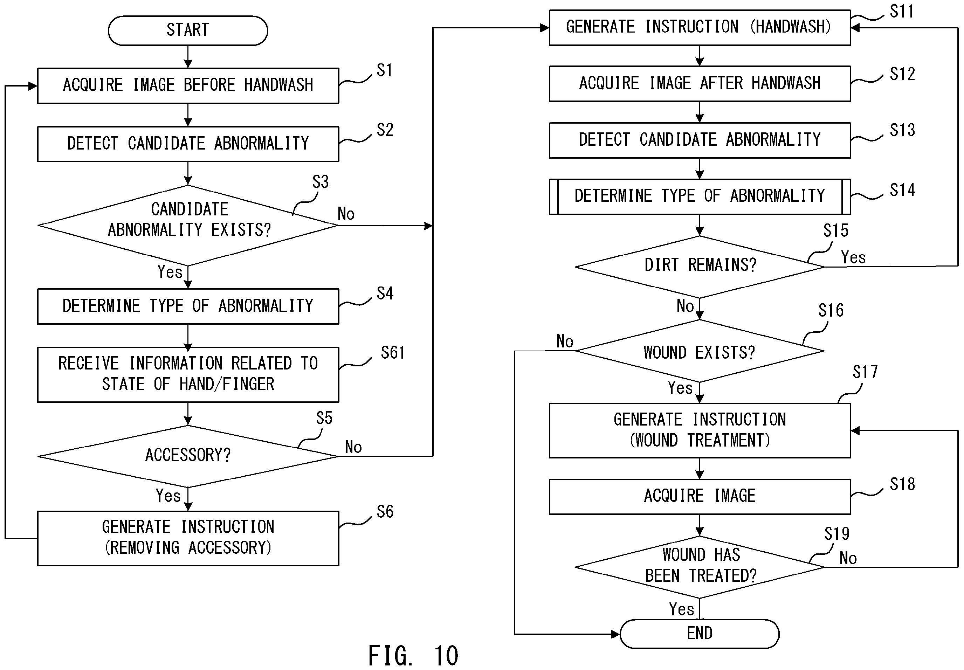

FIG. 10 is a flowchart illustrating a third variation of the handwash monitoring method. Note that, in the third variation, in addition to S1-S6 and S11-S19 illustrated in FIG. 2, S61 is performed. S61 is performed after S4 in this example. Specifically, S61 is performed when the type of the abnormality is determined based on the input image.

In S61, the abnormality type determination unit 22 receives an input from the user. The user enters information related to the state of his or her hand/finger. For example, when a hand/finger has a wound, the user may enter information representing the location of the wound. If a candidate abnormality is detected by the candidate abnormality detector 21, the user may enter information representing an attribute (dirt, wound, mole, scar, tattoo, or others) of the candidate abnormality. Further, if the type determined by the abnormality type determination unit 22 in S4 is incorrect, the user may correct the determination result.

As described above, in the third variation, the information related to the type of the abnormality is entered or corrected by the user himself/herself. The information entered or corrected by the user is used when the handwash flow to be performed by the user is determined. Therefore, even if it is difficult to determine the type of the abnormality only by image recognition, it is possible to provide an appropriate handwash instruction to the user.

### Variation 4

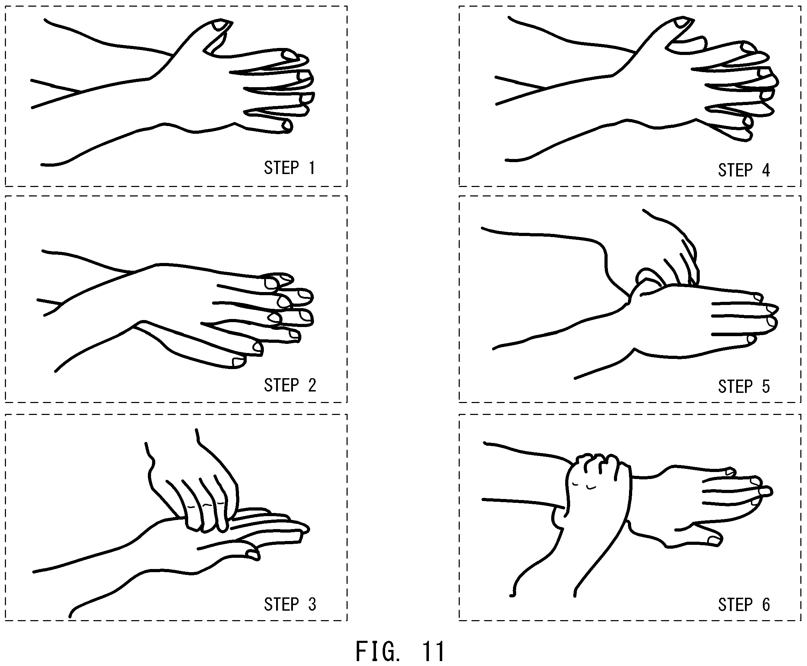

In a fourth variation, the handwash monitoring system 100 monitors a handwash operation of a person and determines whether the handwash operation is correctly performed. It is assumed that the handwash operation monitored by the handwash monitoring system 100 includes a plurality of previously determined operation steps. Specifically, although not particularly limited, the handwash operation includes operation steps 1-6 illustrated in FIG. 11. Note that the operation steps 1-6 are as follows.
Step 1: Wet the palms with running water and then rub the palms together with detergent.
Step 2: Rub the back of the other hand with one palm.
Step 3: Wash between each fingertip and each nail.
Step 4: Wash between each finger.
Step 5: Wash the thumb and its base while twisting.
Step 6: Wash the wrist.

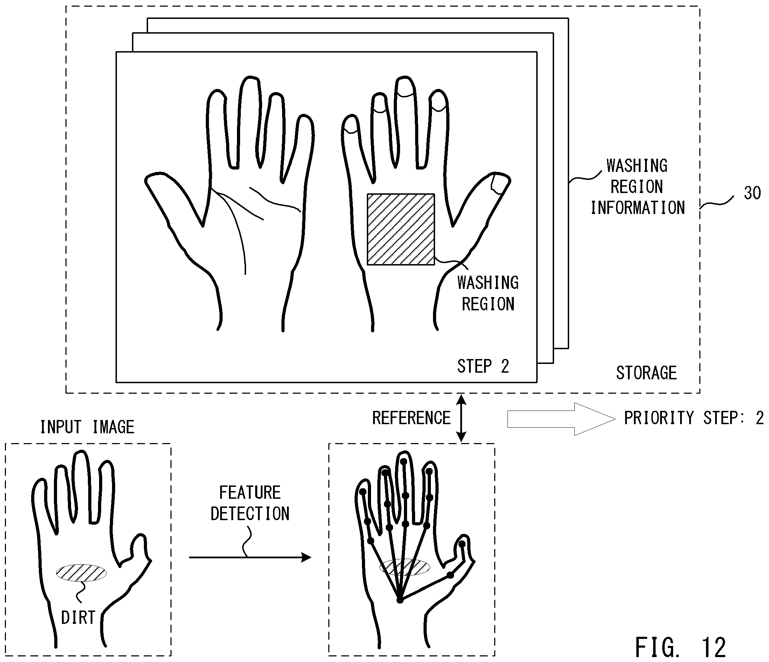

The storage 30 stores cleaning region information. The cleaning region information represents a region to be cleaned in each operation step. In the example illustrated in FIG. 12, the cleaning region information represents that the back of the hand is cleaned in the operation step 2.

Although not illustrated in FIG. 1, the handwash monitoring system 100 includes a priority step determination unit. The priority step determination unit is realized by the processor 20 executing a handwash monitoring program. As illustrated in FIG. 12, the priority step determination unit specifies the position of the candidate abnormality detected by the candidate abnormality detector 21 by acquiring the feature information of the hand region in the input image. In the example illustrated in FIG. 12, it is recognized that a candidate abnormality (e.g., dirt) exists on the back of the left hand.

The priority step determination unit determines a priority step by referring to the cleaning region information. In this example, the operation step of cleaning the back of the hand (i.e., operation step 2) is specified as the priority step. The priority step determination unit may specify the priority step by projecting the candidate abnormality to the standard hand space of the cleaning region information based on the feature of the hand region. The priority step determination unit then notifies the handwash instruction generator 23 of the specified priority step. Thus, the handwash instruction generator 23 gives the user an instruction for carefully performing the notified priority step. The handwash monitoring system 100 may then make a criterion for determining whether the priority step is correctly performed stricter than other operation steps.

The priority step determination unit may output the following proposal.
(1) If the candidate abnormality is dirt, the administrator visually checks that the dirt has been sufficiently removed.
(2) If the candidate abnormality is a wound, the administrator checks the state of the wound at a later date.

As described above, in the fourth variation, an important step is determined from among a plurality of handwash operation steps before the user performs handwashing, and is notified to the user. Therefore, the user can reliably perform important operation steps.

Note that, in the example described above, the priority step is specified before the user performs handwashing, but the fourth variation is not limited to this sequence. For example, if it is determined that dirt remains in S15 illustrated in FIG. 2, the priority step determination unit may specify an operation step for removing the dirt. In this case, the priority step determination unit detects the region of the dirt remaining in the hand region. The priority step determination unit then determines an operation step having a cleaning region including the dirty region. According to this method, the user can be notified of an appropriate operation step for dirt remaining after handwashing.

### Variation 5

The handwash monitoring system 100 matches the position and angle of the hand region and the posture of the fingers with the reference hand/finger image of a target person by using the feature information of the hand region in the input image. The feature information includes the posture of the hand (the position of the joint of each finger and the position of the tip of each finger), the contour of the hand, and the wrinkle of the hand.

After the position alignment, the handwash monitoring system 100 specifies an image region corresponding to a candidate abnormality (a wound, dirt, an accessory, or others) based on a difference between the two images. It is preferable to perform pre-processing for adjusting white balance, contrast, brightness and others. A difference may be calculated by deep learning.

The handwash monitoring system 100 determines the attribute (wound, dirt, watch, ring, nail polish, or others) of each candidate abnormality. The attribute may be determined based on the position of the candidate abnormality. For example, a candidate abnormality appearing at the base of a finger is estimated to be a ring, and a candidate abnormality appearing at the wrist is estimated to be a wristwatch. Based on the change between the image before handwashing and the image after handwashing, a wound and dirt are identified. For example, if there is no change in the shape of the candidate abnormality between the two images, the candidate abnormality is estimated to be a wound, and if there is a change between the two images, the candidate abnormality is estimated to be dirt. The wound and dirt may be identified based on the color of the candidate abnormality.

### Variation 6

The handwash monitoring system 100 may measure the length of the nail when the user washes the hand. If the length of the nail is inappropriate (including too long and too short), the handwash monitoring system 100 outputs an alert.

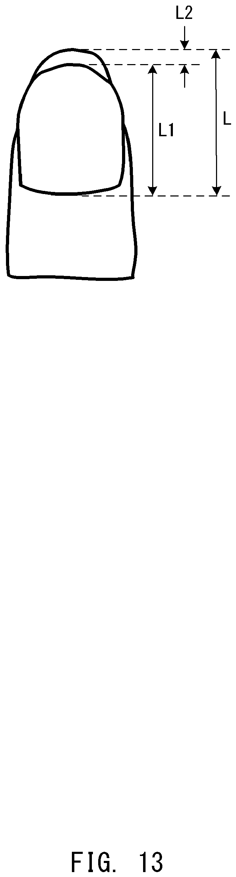

The handwash monitoring system 100 calculates the length of each finger nail of the user using the input image. In the example illustrated in FIG. 13, a length L is calculated. The length of the nail when the nail is in an ideal state is registered in advance for each user. The handwash monitoring system 100 then calculates a difference between the length L obtained from the input image and the ideal length, and compares the difference with a specified threshold. Thus, if the difference is larger than the threshold, the handwash monitoring system 100 outputs an alert to the user.

The handwash monitoring system 100 may determine the state of the nail by using a nail plate length L1 and a nail tip length L2 illustrated in FIG. 13. In this case, for example, if (L1+L2)/L1 is out of a specified threshold range, an alert is output. According to this method, it is possible to determine whether the length of the nail is appropriate even for a user whose ideal nail state is not registered.

### Variation 7

If it is determined that the hand/finger of the user has a wound, the handwash monitoring system 100 may estimate whether the state of the wound is improved or deteriorated in comparison with the past data. For example, if the region corresponding to the wound is widened or if the number of wounds is increased, it is estimated that the state of the wound is deteriorated.

If a wound detected in the past is not detected in a new input image, a period from the time when the wound is detected to the present time is calculated. If this period is shorter than the period estimated to be required for wound healing, the handwash monitoring system 100 performs the following process .
(1) Confirm with the person whether the wound has healed.
(2) Ask the administrator to confirm whether the wound has healed.
(3) Perform photographing so that the relevant portion can be seen more clearly, and perform wound detection process again.

### Variation 8

The handwash monitoring system 100 may include a biometric information acquisition device to identify a user or an administrator thereof. In this case, the handwash monitoring system 100 performs, for example, palm vein authentication or face authentication. When each user or each administrator carries a personal authentication IC chip, the handwash monitoring system 100 may have a function of reading the personal authentication IC chip.

### Variation 9

The handwash monitoring system 100 may have a function of receiving information representing a situation of the user. For example, the user enters information representing his or her situation ("before start of work", "immediately after going to a toilet" , or others) by using this function. Thus, the handwash monitoring system 100 may change the handwash flow in response to the information input from the user. For example, if the information is "immediately after going to a toilet", the handwash monitoring system 100 may instruct the user to increase the number of times of rubbing his or her hand.

### Variation 10

The handwash monitoring system 100 may have a function of recording a handwash monitoring result for each user. For example, the presence or absence of a wound, the position, size, type, and others of the wound are recorded. It is recorded that a treatment for the wound (sticking of an adhesive plaster, wearing of a vinyl glove, or others) has been performed. The presence or absence of dirt/accessory and the position, size and type of the dirt/accessory may be recorded. In addition, it is recorded after the end of the handwashing that dirt has been removed.

In a case where the data of each user is recorded as described above, the handwash monitoring system 100 may adjust a parameter related to handwash monitoring by using the data of a plurality of users. For example, the handwash monitoring system 100 may adjust a parameter for detecting a candidate abnormality and a parameter for determining the type of the candidate abnormality.

### Variation 11

The handwash monitoring system 100 may have a function of recognizing a shape and/or movement of a hand of a user. In this case, a previously determined shape or movement is captured by the imaging device 10. Thus, the handwash monitoring system 100 performs a process corresponding to the shape or the movement. This configuration allows the user to give an instruction to the handwash monitoring system 100 in a non-contact manner even if the hand/finger is dirty.

### Variation 12

The handwash monitoring system 100 may have a function of detecting dirt on a lens of the imaging device 10. For example, the storage 30 stores a reference image when the hand/finger of the user is not within the imaging range. In this case, the handwash monitoring system 100 acquires an image captured by the imaging device 10 before the handwash monitoring process is started (i.e., before the user positions his or her hand/finger within the imaging range). Dirt on the lens is detected by comparing the acquired image with the reference image.

When dirt on the lens is detected, the handwash monitoring system 100 asks the administrator or the user to remove the dirt on the lens. The handwash monitoring system 100 then performs a previously determined confirmation procedure, and confirms whether the dirt on the lens has been removed.

### Variation 13

The handwash monitoring system 100 may include a video projector that optically projects a video into space. In this case, the video projector projects an alert video around the detected wound, dirt, accessory, or others. The video projector may project a video representing how to deal with the detected wound, dirt, and accessory. Further, the handwash monitoring system 100 may use a video projector to project a video for guiding the correct hand position.

Reference Sign List
- 10: Imaging device
- 20: Processor
- 21: Candidate abnormality detector
- 22: Abnormality type determination unit
- 23: Handwash instruction generator
- 30: Storage
- 100: Handwash monitoring system

## Claims

1. A handwash monitoring system comprising:
an imaging unit (10);
a detector (21) configured to detect a first candidate abnormality existing in a hand of a user from a first image captured by the imaging unit before handwashing, and detect a second candidate abnormality existing in the hand of the user from a second image captured by the imaging unit after the handwashing; and
an abnormality type determination unit (22) configured to determine a type of an abnormality on the hand of the user based on a comparison between the first candidate abnormality and the second candidate abnormality.

2. The handwash monitoring system according to claim 1, further comprising
an instruction generator (23) configured to generate an instruction related to handwashing based on a type of an abnormality determined by the abnormality type determination unit.

3. The handwash monitoring system according to claim 1, further comprising
a storage (30) configured to store a reference image representing a past state of the hand of the user, wherein
the detector detects the first candidate abnormality by comparing the reference image with the first image.

4. The handwash monitoring system according to claim 1, further comprising
a storage (30) configured to store a reference image representing a past state of the hand of the user, wherein
the detector detects the second candidate abnormality by comparing the reference image with the second image.

5. The handwash monitoring system according to claim 2, wherein
when the first candidate abnormality and the second candidate abnormality are detected from an identical region and a shape of the first candidate abnormality and a shape of the second candidate abnormality are different from each other, the instruction generator instructs the user to further perform handwashing.

6. The handwash monitoring system according to claim 5, wherein
the instruction generator instructs the user to change a detergent for washing hands.

7. The handwash monitoring system according to claim 5, wherein
the instruction generator instructs the user on a handwash operation to be performed by the user based on a position of the second candidate abnormality.

8. The handwash monitoring system according to claim 2, wherein
the instruction generator instructs the user on a handwash operation flow based on a color component of the first candidate abnormality or the second candidate abnormality.

9. The handwash monitoring system according to claim 2, wherein
when the first candidate abnormality and the second candidate abnormality are detected from an identical region and a shape of the first candidate abnormality and a shape of the second candidate abnormality are identical or substantially identical to each other, the abnormality type determination unit determines that the second candidate abnormality is a wound on the hand of the user, and
the instruction generator instructs the user to perform treatment of the wound.

10. The handwash monitoring system according to claim 2, wherein
the abnormality type determination unit determines whether the first candidate abnormality is an accessory based on a position and a shape of the first candidate abnormality, and
when it is determined that the first candidate abnormality is an accessory, the instruction generator instructs the user to remove the accessory.

11. The handwash monitoring system according to claim 2, further comprising
a storage (30) configured to store a reference image representing a specified accessory, wherein
the abnormality type determination unit determines whether the first candidate abnormality is an accessory by comparing the first image with the reference image, and
when it is determined that the first candidate abnormality is an accessory, the instruction generator instructs the user to remove the accessory.

12. A handwash monitoring system comprising:
an imaging unit (10);
a storage (30) configured to store a reference image representing a past state of a hand of a user;
a detector (21) configured to detect a first candidate abnormality existing in the hand of the user from the reference image, and detect a second candidate abnormality existing in the hand of the user from an image captured by the imaging unit after handwashing; and
an abnormality type determination unit (22) configured to determine a type of an abnormality on the hand of the user based on a comparison between the first candidate abnormality and the second candidate abnormality.

13. A handwash monitoring method comprising:
detecting a first candidate abnormality existing in a hand of a user from a first image captured by an imaging device before handwashing;
detecting a second candidate abnormality existing in the hand of the user from a second image captured by the imaging device after the handwashing; and
determining a type of an abnormality on the hand of the user based on a comparison between the first candidate abnormality and the second candidate abnormality.

14. A handwash monitoring program for causing a processor to execute a process, the process comprising:
detecting a first candidate abnormality existing in a hand of a user from a first image captured by an imaging device before handwashing;
detecting a second candidate abnormality existing in the hand of the user from a second image captured by the imaging device after the handwashing; and
determining a type of an abnormality on the hand of the user based on a comparison between the first candidate abnormality and the second candidate abnormality.
